(19) Europäisches Patentamt
European Patent Office
Office européen des brevets

(11) **EP 0 561 722 B1**

(12) **FASCICULE DE BREVET EUROPEEN**

(45) Date de publication et mention de la délivrance du brevet:
**16.10.1996 Bulletin 1996/42**

(51) Int Cl.⁶: **C08F 8/30**

(21) Numéro de dépôt: **93420122.9**

(22) Date de dépôt: **17.03.1993**

(54) **Composés hydrosolubles dérivés d'un homopolymère ou copolymère de l'anhydride maléique, et applications desdits composés au support de molécules biologiques**

Wasserlösliche Verbindungen aus Maleinsäureanhydridpolymeren und Copolymeren, und Verwendung dieser Verbindungen zu Trägern für biologische Moleküle

Water-soluble compounds from polymers and copolymers of maleic anhydride, and use of these compounds as carriers for biological molecules

(84) Etats contractants désignés:
**BE CH DE ES FR GB IT LI SE**

(30) Priorité: **17.03.1992 FR 9203425**

(43) Date de publication de la demande:
**22.09.1993 Bulletin 1993/38**

(73) Titulaire: **BIO MERIEUX**
**F-69280 Marcy l'Etoile (FR)**

(72) Inventeurs:
• **Charles, Marie-Hélène**
  **F-69420 Condrieu (FR)**
• **Delair, Thierry**
  **F-69007 Lyon (FR)**
• **Jaubert, Monique**
  **F-69290 Craponne (FR)**
• **Mandrand, Bernard**
  **F-69100 Villeurbanne (FR)**

(74) Mandataire: **Guerre, Dominique et al**
**Cabinet Germain et Maureau**
**20 Boulevard Eugène Deruelle**
**BP 3011**
**69392 Lyon Cédex 03 (FR)**

(56) Documents cités:
EP-A- 0 273 895          DE-A- 1 745 954
DE-A- 2 420 747          FR-A- 1 580 038
FR-A- 2 384 811          US-A- 3 310 540

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne un composé hydrosoluble d'un homopolymère ou copolymère de l'anhydride maléique, un agent d'hydrophilisation d'un tel homopolymère ou copolymère, un procédé pour préparer ledit composé hydrosoluble et enfin les applications du composé préparé.

Les homopolymères d'anhydride maléique ou copolymères à base d'anhydride maléique non hydrolysés sont insolubles dans l'eau à pH neutre. Ils deviennent peu à peu solubles par réaction d'hydrolyse en milieu aqueux, du fait de l'ouverture des cycles anhydride maléique par attaque nucléophile des molécules d'eau sur les fonctions anhydride et ouverture subséquente des cycles avec production de fonctions carboxyliques.

Une solubilisation directe en milieu aqueux par hydrolyse des fonctions anhydride présente l'inconvénient d'être un processus long et difficilement contrôlable. En effet, on ne sait pas combien de fonctions anhydride sont. consommées pour "dissoudre" le polymère.

On sait par ailleurs que les homopolymères ou copolymères d'anhydride maléique sont solubles dans certains solvants organiques.

B. SOLOMON et Y. LEVIN (Biotechnology and Bioengineering, vol. XVI, pages 1161-1177 (1974)) ont décrit un procédé de couplage d'une enzyme à des copolymères éthylène-anhydride maléique, selon lequel le copolymère d'anhydride maléique dissous dans de l'acétone à une concentration de 10 % (poids/volume) est mélangé à une préparation enzymatique, pour la fixation de cette dernière sur le copolymère. Par effet compétitif entre les molécules d'eau de la préparation enzymatique et les groupements aminés de l'enzyme, on obtient une hydrolyse du copolymère permettant à la fois sa dissolution et la fixation de la fraction protéique. Cependant, comme expliqué dans cette publication, il se forme un précipité partiel du copolymère éthylène-anhydride maléique au cours du mélange de la solution organique contenant le copolymère et de la solution protéique, ce qui nécessite une étape supplémentaire d'agitation pendant au moins une nuit, jusqu'à l'obtention de la dissolution complète du précipité. Ce procédé n'est donc pas entièrement satisfaisant car il présente des difficultés techniques de mise en oeuvre dûes à la formation de ce précipité.

Une solution pour pallier ce problème pourrait être trouvée dans la publication de Léon GOLDSTEIN et al. (Int. J. Biochem. 2,448,1971, Water-insoluble dérivatives of naringinase) qui consiste à mélanger le copolymère d'anhydride maléique dans une solution contenant environ 50 à 70 % d'un solvant organique tel que l'acétone, le diméthylformamide (DMF) ou le diméthylsulfoxide (DMSO) pour la dissolution complète du copolymère et à ajouter la solution de copolymère d'anhydride maléique ainsi obtenue, à une solution aqueuse d'une enzyme pour la fixation de cette dernière sur le copolymère. Une phase homogène est effectivement obtenue.

Cependant, cette solution est particulièrement désavantageuse quand l'enzyme ou toute autre molécule biologique à fixer sur l'homopolymère ou copolymère est sensible au solvant organique, risquant d'être partiellement voire totalement dénaturée et, de ce fait, perdre toute activité biologique.

Selon DE-A-24 20 747, il est décrit un composé hydrosoluble dérivé d'un copolymère anhydride maléique/éthylène destiné à immobiliser des enzymes.

Ce dérivé présente des fonctions anhydride hydrolysées qui consistent en des fonctions carboxyliques portant un résidu d'une amine choisie parmi l'hydrazine, le p,p'-diamino-diphényl-méthane (MDA) et le diamino-1,6-hexane. La fixation de l'une ou l'autre de ces amines sur ledit copolymère lui confère une certaine solubilité en phase aqueuse, permettant ainsi d'effectuer l'étape de fixation de l'enzyme sur ledit dérivé en milieu aqueux. Cependant, avant l'étape de fixation, une étape supplémentaire d'activation des groupements anhydride transformés est nécessaire.

La présente invention permet de résoudre les problèmes posés par les solutions actuellement existantes, en apportant un composé hydrosoluble dérivé d'un homopolymère ou copolymère d'anhydride maléique, directement utilisable pour immobiliser une molécule biologique, sans qu'une étape préalable d'activation ne soit nécessaire.

Selon l'invention, ce composé présente des fonctions anhydride disponibles ou libres et des fonctions anhydride hydrolysées, les fonctions anhydride hydrolysées consistant en des fonctions carboxyliques et des fonctions dérivées de fonctions carboxyliques portant un résidu d'un composé répondant à la formule I :

$$\text{Cx Hy Az Ot} \tag{I}$$

dans laquelle :

- A est l'atome d'azote d'une fonction amine primaire, secondaire ou tertiaire ou l'atome de soufre d'une fonction thiol,
- x, z et t indépendamment les uns des autres sont des nombres entiers non nuls, et
- y est un nombre entier non nul, au moins égal à 5 quand A est un atome d'azote, et au moins égal à 4 quand A est un atome de soufre.

Les fonctions dérivées de fonctions carboxyliques sont notamment choisies parmi les fonctions ester, amide, thiocarboxy.

Quand A est l'atome d'azote, le composé I est avantageusement choisi parmi le tris(hydroxyméthyl) aminométhane, l'(amino-2-éthylamino)-2-éthanol, l'amino-2-méthyl-2-propanediol-1,3, la diéthanolamine, les polypeptides et de préférence une polytyrosine ou polyglycine, le N-BOC-1,6-diaminohexane et les composés répondant aux formules générales respectivement :

$$(II) \qquad HO\text{-}(CH_2)n\text{-}NH_2$$

$$(III) \qquad HO\text{-}(CH_2\text{-}CH_2)_n\text{-}O\text{-}(CH_2\text{-}CH_2)n\text{-}NH_2$$

$$(IV) \qquad HO\text{-}(CH_2\text{-}CH_2\text{-}O)n\text{-}CH_2\text{-}CH_2\text{-}NH_2$$

dans lesquelles n est un nombre entier compris entre 1 et 50.

De préférence n est compris entre 1 et 10 et le composé II est choisi parmi l'éthanolamine et l'(amino-2-éthoxy)-2-éthanol.

Quand A est l'atome de soufre, le composé I est avantageusement le mercaptoéthanol.

Les composés hydrosolubles de l'invention sont des dérivés de copolymères de l'anhydride maléique, choisis parmi les poly (anhydride maléique-éthylène), les poly (anhydride maléique-styrène), les poly (anhydride maléique-propylène), les poly (anhydride maléique-méthylvinyléther).

De préférence, les copolymères comprennent au moins 5 % de motifs anhydride maléique.

Le composé hydrosoluble est utilisé selon l'invention pour immobiliser au moins une molécule biologique, à laquelle il est lié directement ou indirectement. Ladite molécule biologique est notamment choisie parmi des protéines telles que des anticorps ou des fragments d'anticorps ou des antigènes; des polypeptides; des enzymes; des petites molécules telles que des haptènes; des fragments d'acides nucléiques.

Le terme "fragment d'acide nucléique" tel qu'utilisé dans la présente invention, signifie un fragment d'ADN ou d'ARN naturel, ou un oligonucléotide naturel ou de synthèse, ou un fragment d'ADN ou d'ARN de synthèse non modifié ou comprenant une ou plusieurs bases modifiées telles que l'inosine, la méthyl-5-désoxycytidine, la diméthylamino-5-désoxyuridine, la désoxyuridine, la diamino-2,6-purine, la bromo-5-désoxyuridine ou toute autre base modifiée.

Ainsi pour immobiliser des protéines, le composé hydrosoluble de l'invention est ajouté à une solution protéique, les groupements aminés des protéines réagissant pour former des liaisons covalentes avec le composé dérivé de l'homopolymère ou copolymère, sur les fonctions anhydride encore disponibles, c'est-à-dire les fonctions anhydride non hydrolysées.

Selon un mode d'utilisation particulier du composé de l'invention, ce dernier est fonctionnalisé par interaction des fonctions anhydride disponibles, avec des fonctions réactives d'un agent de fonctionnalisation, lesdites fonctions réactives étant notamment choisies parmi les fonctions amine, thiol et hydroxyle.

Au sens de la présente invention, une fonctionnalisation est une modification chimique de l'homopolymère ou copolymère d'anhydride maléique par un agent de fonctionnalisation, en vue de :

1) son immobilisation directe ou indirecte sur un support solide : par immobilisation directe, on entend la fixation par covalence ou adsorption passive de l'homopolymère ou copolymère sur ledit support solide préalablement fonctionnalisé ou non ; une immobilisation directe peut être réalisée au moyen d'un ligand, préalablement fixé sur l'homopolymère ou copolymère, puis fixé chimiquement sur ledit support solide ; par immobilisation indirecte, on entend l'interaction ligand/antiligand entre un ligand fixé sur l'homopolymère ou copolymère, et l'antiligand ou ligand complémentaire fixé sur le support solide.
2) son marquage pour le détecter
3) la fixation indirecte d'une molécule biologique sur ledit homopolymère ou copolymère.

L'agent de fonctionnalisation est notamment choisi parmi les haptènes, des agents colorants, luminescents, fluorescents ou phosphorescents, et des polymères naturels ou synthétiques notamment polypeptides, polysaccharides et protéines solubles en milieu organique et le N-BOC-1,6-diaminohexane.

Selon l'invention, il ressort que si la liaison entre le composé de l'invention et la ou les molécules biologiques est indirecte, le composé est lié à un agent de fonctionnalisation pouvant appartenir au groupe des agents cités ci-dessus, l'agent de fonctionnalisation étant lui-même lié à la ou les molécules biologiques.

En outre, le composé portant la ou les molécules biologiques, fonctionnalisé ou non, peut être lié directement ou indirectement à un support solide. Le composé peut être directement lié sur un support solide par un ligand notamment choisi parmi les polypeptides et la polytyrosine et la polyglycine et le N-BOC-1,6-diaminohexane. Quand la liaison entre le composé et le support solide est indirecte, le composé est lié à un agent de fonctionnalisation qui est lié à un agent de fonctionnalisation complémentaire, ce dernier étant lié à un support solide.

Selon l'invention, le composé répondant à la formule I peut être à la fois, l'agent d'hydrophilisation et l'agent de fonctionnalisation, et est de préférence un polypeptide choisi parmi la polytyrosine et la polyglycine ou le N-BOC-1,6-diaminohexane.

Avantageusement, le support solide est choisi parmi les matériaux naturels, de synthèse modifiés ou non chimiquement, et notamment parmi les latex, les polymères du type polychlorure de vinyle, polyéthylène, polystyrène, polyacrylate et les copolymères du type de ceux à base de styrène.

Le support solide peut être sous la forme d'une plaque de microtitration, d'un cône, d'un tube, d'un puits, de billes ou analogues.

La présente invention concerne également un agent d'hydrophilisation d'un homopolymère ou copolymère de l'anhydride maléique, cet agent répondant à la formule I :

$$Cx\ Hy\ Az\ Ot \hspace{4cm} (I)$$

dans laquelle :

- A est l'atome d'azote d'une fonction amine primaire, secondaire ou tertiaire ou l'atome de soufre d'une fonction thiol,
- x, z et t indépendamment les uns des autres sont des nombres entiers non nuls, et
- y est un nombre entier non nul, au moins égal à 5 quand A est un atome d'azote, et au moins égal à 4 quand A est un atome de soufre.

L'agent hydrophilisant est un composé soluble à la fois dans l'eau et en milieu organique, capable de se fixer sur l'homopolymère ou copolymère d'anhydride maléique pour lui conférer une totale solubilité en phase majoritairement aqueuse.

Un autre objet de l'invention est un procédé pour obtenir un dérivé soluble en phase aqueuse à pH neutre, d'un homopolymère ou copolymère d'anhydride maléique consistant :

- à dissoudre l'homopolymère ou copolymère dans un solvant organique anhydre et
- à faire réagir l'homopolymère ou copolymère en solution, avec l'agent hydrophilisant de l'invention, en phase organique.

Pour la mise en oeuvre de ce procédé, et en particulier pour l'étape de la dissolution, le solvant employé est de préférence de type aprotique polaire tel que le diméthylformamide (DMF), le diméthylsulfoxide (DMSO), la pyridine, la 1,3-diméthyl -3,4,5,6 tétrahydro-2 (1H)-pyrimidinone, le dioxane, ou analogues.

La quantité de solvant à employer dépend de la nature chimique de l'homopolymère ou copolymère, de son poids moléculaire et de la nature du solvant organique. Aussi pour deux polymères de poids moléculaire identique, le polymère le plus hydrophobe se dissoudra plus facilement dans le solvant organique. La quantité de solvant est au moins égale à la quantité nécessaire pour dissoudre l'homopolymère ou copolymère à température ambiante, mais peut être supérieure sans pour autant nuire au bon déroulement du procédé.

Enfin, un dernier objet de l'invention est un procédé pour immobiliser une molécule biologique sur un homopolymère ou copolymère de l'anhydride maléique consistant :

- à dissoudre l'homopolymère ou copolymère dans un solvant organique anhydre et
- à faire réagir l'homopolymère ou copolymère dissous avec un agent hydrophilisant selon l'invention, en phase organique et
- à mettre en contact le composé hydrosoluble obtenu avec la molécule biologique, en phase aqueuse.

Le procédé d'immobilisation de l'invention peut en outre comprendre, avant la mise en contact du composé hydrosoluble avec la molécule biologique, une étape de fonctionnalisation :

- de l'homopolymère ou copolymère d'anhydride maléique, quand elle est effectuée avant l'étape d'hydrophilisation dudit homopolymère ou copolymère
- ou du composé hydrosoluble, quand elle est effectuée après l'étape d'hydrophilisation de l'homopolymère ou copolymère.

L'étape de fonctionnalisation est effectuée en phase organique avec un agent de fonctionnalisation qui est avantageusement choisi parmi les haptènes, des agents colorants, luminescents, fluorescents ou phosphorescents et des polymères naturels ou synthétiques notamment polypeptides, polysaccharides et protéines solubles en milieu organique, et le N-BOC-1,6-diaminohexane.

La réaction de fonctionnalisation a lieu à des températures comprises entre + 4°C et + 50°C, préférentiellement entre + 4°C et + 37°C, et plus couramment à température ambiante. Le temps de contact entre le ou les agents de fonctionnalisation et l'homopolymère ou copolymère s'échelonne entre 30 mn et 48 heures, préférentiellement entre une heure et 24 heures. Une agitation du milieu réactionnel n'est pas toujours indispensable.

Les différents objets de l'invention ainsi que leurs caractéristiques et avantages sont à présent illustrés par les exemples 1 à 6 suivants :

## EXEMPLE 1

Préparation de composés hydrosolubles dérivés de copolymères de l'anhydride maléique

Le Tableau 1 suivant fait apparaître les conditions de préparation d'un composé hydrosoluble dérivé de trois copolymères de l'anhydride maléique respectivement A, B et C :

Copolymère A commercialisé par la Société POLYSCIENCE sous la référence 3106

Copolymère B commercialisé par la Société JANSSEN sous la référence 17.923 -77

Copolymère C commercialisé par la Société POLYSCIENCE sous la référence 3102.

Selon le procédé de l'invention, le copolymère est dissous dans un solvant organique anhydre, le diméthylformamide (DMF) anhydre, puis l'agent hydrophilisant, l'éthanolamine, est additionné en milieu organique. La quantité à employer d'agent hydrophilisant en général et d'éthanolamine en particulier pour cet exemple, dépend de la nature chimique du polymère ou copolymère, de la concentration du polymère ou copolymère dans la phase organique et du volume de la phase aqueuse. La quantité d'agent hydrophilisant ajoutée dépend essentiellement de l'hydrophilie intrinsèque du polymère ou copolymère.

Ainsi, le Tableau 1 donne les quantités d'éthanolamine nécessaire pour que 100 µl de solution de composé hydrosoluble dérivé des copolymères respectivement A, B et C, dilués dans 900 µl de milieu aqueux, fournissent une solution homogène.

TABLEAU 1

| Copolymère 50/50 anhydride maléique / P | A | B | C |
|---|---|---|---|
| P | styrène | styrène | méthylvinyléther |
| Mw (masse moléculaire) | 1600 | 50 000 | 67 000 |
| quantité de copolymère (mg) | 100 | 50 | 15 |
| DMF (ml) | 1 | 1 | 1 |
| éthanolamine (µl) | 15 | 7,5 | 1 |
| % des fonctions anhydride consommées par l'éthanolamine | 50 | 50 | 25 |

## EXEMPLE 2

Immobilisation d'allergènes sur un composé hydrosoluble dérivé du copolymère anhydride maléique/styrène, B, de l'exemple 1, puis fixation sur des particules de latex fonctionnalisé

A 1 ml d'une solution de B dans du DMF anhydre, on ajoute 7,5 µl d'éthanolamine, puis on agite pendant 30 sec. Dans un tampon carbonate 100 mM pH 8,2, contenant 0,5 mg/ml d'extrait aqueux Dactyle (Laboratoire des Stallergènes) on additionne successivement 4 µl de solution de B diluée au 1/5 dans du DMF et 200 µl de suspension de latex aminé à 5 % de taux de solide (Société BioMérieux) pour obtenir, en final, un volume total de 1 ml. Après agitation douce pendant 18 heures, le latex est centrifugé 5 mn à 8000 tpm dans une centrifugeuse Eppendorf. Le culot est repris dans un tampon glycine 0,1M ; NaCl 0,15 M ; pH8,2 ; BSA 5 g/l.

* Test d'activité des allergènes immobilisés :

Dans une plaque filtrante de microtitration, (Société Pall) on dépose dans chaque puits 100 µl de suspension de latex et 50 µl de sérum. Après 3 heures d'incubation à température ambiante, on procède à 2 lavages en PBS-Tween par filtration sous vide. On introduit ensuite 100 µl d'anticorps anti-IgE marqué à la peroxydase de raifort et on laisse incuber 1 heure à température ambiante avant de laver deux fois. Ensuite, on ajoute 250 µl de substrat ortho-phénylènediamine. Après 30 mn d'incubation, on bloque la réaction enzymatique par addition de 100 µl de $H_2SO_4$, on filtre dans une plaque de microtitration et la densité optique du filtrat est lue sur un lecteur de plaques, à la longueur d'onde de 492 nm.

Les résultats (exprimés en unité de D.O.) sont présentés dans le Tableau 2 suivant :

**TABLEAU 2**

|  | SERUM NEGATIF | SERUM POSITIF |
|---|---|---|
| Témoin LATEX sans copolymère | 0,056 | 0,387 |
| Allergènes immobilisés sur copolymère fixé sur LATEX | 0,064 | 1,050 |

Le système présente de faibles bruits de fond et le signal spécifique est élevé.

## EXEMPLE 3

Immobilisation d'allergènes sur un composé dérivé du copolymère anhydride maléique/styrène, B, de l'exemple 1, puis fixation dudit dérivé sur un support solide

* Fonctionnalisation par la biotine puis hydrophilisation du copolymère B

On ajoute 1 ml de solution de B dans du DMF anhydre à 1 mg de biotine hydrazide (Pierce) et on laisse agir deux heures à température ambiante. Le copolymère est utilisable tel quel sans aucune étape de purification.

A la solution de copolymère fonctionnalisé, on ajoute 7,5 µl d'éthanolamine en tant qu'agent hydrophilisant.

* Immobilisation d'allergènes sur la partie non fonctionnalisée du dérivé du copolymère B préparé ci-dessus :

Ensuite on dilue le copolymère ainsi obtenu au 1/10 dans le DMF et l'on ajoute 2,5 µl de mélange copolymère-biotine à 0,5 ml de solution d'allergènes, extrait Dactyle (Laboratoire des Stallergènes) à 0,5 mg/ml en taux de protéines. Après 4 heures d'incubation à température ambiante, on dialyse 18 heures à + 4°C contre un tampon phosphate 20 mM pH 7,4.

* Fixation de la partie fonctionnalisée du dérivé du copolymère B sur un support solide

* Test d'activité des allergènes immobilisés :

Après deux lavages en PBS Tween, on ajoute 50 µl de sérum et on incube 2 heures à température ambiante. Après 2 nouveaux lavages, on ajoute 100 µl d'anticorps anti-IgE marqué à la peroxydase, et on incube une heure à la même température. Après lavage, la réaction enzymatique a lieu pendant 30 min à température ambiante. Après blocage par 1 ml d'acide sulfurique, la lecture est effectuée à 492 nm.

Les résultats exprimés en unité de densité optique sont les suivants :

SERUM NEGATIF : 0,014
SERUM POSITIF : 1,533

Le système ainsi obtenu présente un très faible bruit de fond et un bon signal spécifique.

## EXEMPLE 4

Immobilisation d'allergènes sur un copolymère anhydride maléique/styrène fonctionnalisé et hydrophilisé par le même agent, la polytyrosine

* Fonctionnalisation du copolymère :

10 mg de copolymère styrène-anhydride maléique de poids moléculaire 360 000 sont dissous dans 1 ml de DMF. On ajoute ensuite 1 ml d'une solution de polytyrosine (Sigma, poids moléculaire 27 000) à 2 mg/ml dans du DMSO. On laisse agir 3 heures à température ambiante. Le copolymère devient suffisamment hydrophile pour être utilisé tel quel dans les conditions de fixation d'allergènes mises au point.

* Immobilisation des allergènes :

A 995 μl de solution d'allergènes à 0,5 mg/ml (extrait Armoise du laboratoire des Stallergènes) dans un tampon carbonate 100 mM pH 8,2, on ajoute 5 μl de solution de copolymère fonctionnalisé, on incube 4 heures à température ambiante.

* Test d'activité des allergènes immobilisés sur le composé hydrosoluble dérivé de B fixé sur cônes :

L'allergène ainsi préparé est testé sur le système VIDAS (Marque déposée BioMérieux) après fixation sur les cônes selon un protocole défini par le fabriquant.
Les résultats exprimés en unités relatives de fluorescence : RFU sont les suivants :

SERUM NEGATIF : 116
SERUM POSITIF :  988

## EXEMPLE 5

Immobilisation d'allergènes sur le composé hydrosoluble dérivé d'un copolymère de l'anhydride maléique fonctionnalisé et hydrophilisé par le même agent, le N-BOC-1,6-diaminohexane

* Fonctionnalisation et hydrophilisation du copolymère :

10 mg de copolymère styrène-anhydride maléique de poids moléculaire 360 000 sont dissous dans 0,7 ml de DMSO. On ajoute ensuite 0,3 ml d'une solution à 3 mg/ml d'hydrochlorure de N-BOC-1,6-diaminohexane (Fluka) dans du DMSO, contenant 10 μl de triéthylamine (Janssen) par ml de solvant. On laisse agir 3 heures à température ambiante. Le polymère devient suffisamment hydrophile pour être utilisé tel quel, ou dilué au demi dans du DMF, dans les conditions de fixation d'allergènes mises au point.

* Immobilisation des allergènes :

A 2490 μl de solution d'allergènes à 0,5 mg/ml (extrait Dactyle du laboratoire des Stallergènes) dans un tampon carbonate 100 mM pH 8,2, on ajoute 12,5 μl de solution de copolymère fonctionnalisé dilué au demi dans du DMF, on incube 4 heures à température ambiante.

* Test d'activité des allergènes immobilisés :

L'allergène ainsi préparé est testé sur le système VIDAS (BioMérieux) après fixation sur les cônes selon un protocole défini par le fabriquant.
Les résultats exprimés en unités relatives de fluorescence : RFU sont les suivants :

SERUM FORTEMENT POSITIF (> 60) :          13 827
SERUM POSITIF (15) :                      3 191
SERUM FAIBLEMENT POSITIF (1,4) :          1 568
SERUM TRES FAIBLEMENT POSITIF (0,44) :    255
SERUM NEGATIF :                           133

Ces méthodes d'immobilisation d'allergènes sur une phase solide selon Exemples 4 et 5 présentent un net avantage par rapport à celle décrite dans l'Exemple 3 en ce que, étant directes, elles ne nécéssitent pas l'emploi d'un anticorps antibiotine. Le protocole est aisé à mettre en oeuvre.

**EXEMPLE 6**

Immobilisation d'anticorps monoclonaux anti-alphafoeto-protéine (anti-AFP) sur le composé hydrosoluble dérivé d'un copolymère de l'anhydride maléique B ou C de l'exemple 1, fonctionnalisé par la biotine, puis fixation sur support solide portant un anticorps anti-biotine

\* Fonctionnalisation du copolymère :

Le copolymère B est fonctionnalisé par la biotine et rendu hydrosoluble selon le prototype décrit dans l'Exemple 3. Le copolymère C est fonctionnalisé et rendu hydrosoluble comme suit :
A 1 ml de solution C, on ajoute 0,1 ml de solution de biotine hydrazide à 2,5 mg/ml. On laisse agir pendant deux heures, après quoi le copolymère fonctionnalisé est traité par l'éthanolamine.

\* Immobilisation des anticorps sur la partie non fonctionnalisée du dérivé du copolymère B ou C préparé ci-dessus :

A une solution d'anticorps à 0,5 mg/ml en final dans un tampon carbonate pH 8,2, 100 mM, on ajoute 5 µl de solution de composé hydrosoluble fonctionnalisé dérivé du copolymère B ou C. On incube 1 heure à 37° C avant de dialyser 4 heures à température ambiante contre du PBS.

\* Fixation de la partie fonctionnalisée du composé dérivé du copolymère B ou C sur un support solide portant un anticorps antibiotine :

Un anticorps antibiotine à raison de 2 µg d'anticorps par puits est fixé sur une plaque de microtitration (NUNC, nom commercial) durant 2 heures à 37° C avant saturation en gélatine 0,5 %. On dépose dans les puits de la plaque ainsi obtenue un volume de solution d'anticorps anti-AFP immobilisés. On laisse 1 heure à 37°C.

\* Test d'activité des anticorps immobilisés :

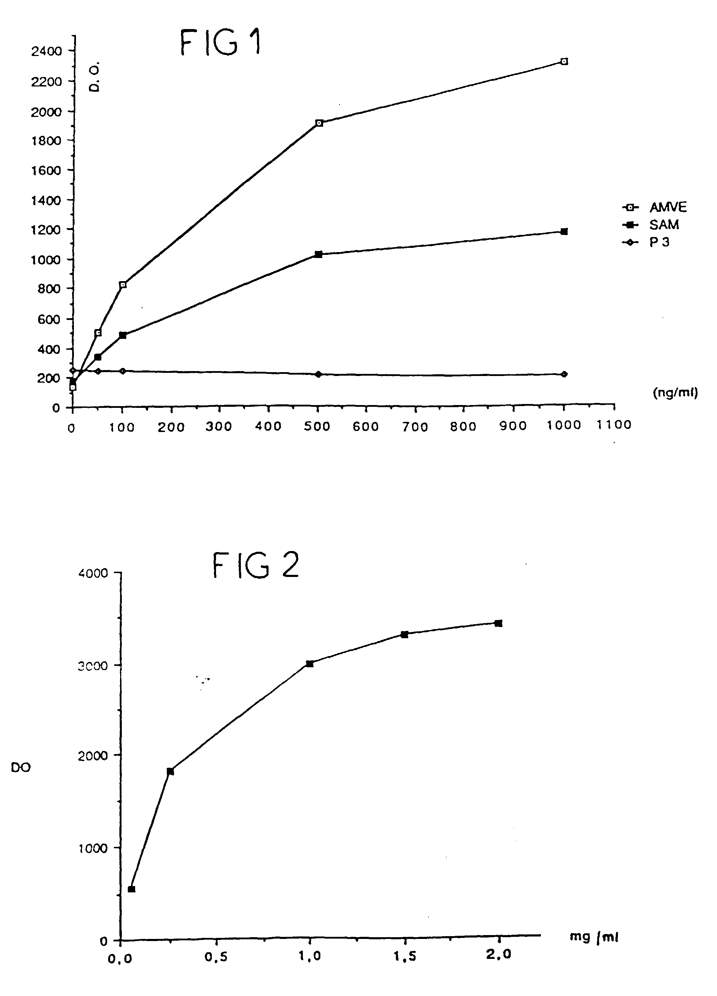

Après 3 lavages en PBS Tween, on introduit une gamme en antigène (AFP). On laisse incuber 1 heure à 37°C, on lave 3 fois en PBS Tween. La présence de l'antigène est détectée par un conjugué phosphatase alcaline - anticorps polyclonal complémentaire. On incube 1 heure à 37°C. La révélation est effectuée par le substrat paranitrophényl phosphate (pNPP). Après blocage avec NaOH 1N, la lecture s'effectue sur un lecteur de plaques, à une longueur d'onde de 405 nm.
Les résultats obtenus sont montrés dans la figure 1. La courbe intitulée P3 est celle de l'anticorps seul. On voit ainsi que ce dernier n'est pas, ou très peu, adsorbé non spécifiquement par la plaque de microtitration revêtue d'anticorps antibiotine.
La courbe intitulée AMVE est celle obtenue avec le copolymère C méthylvinyléther-anhydride maléique. La courbe désignée SAM est celle obtenue en utilisant le copolymère B styrène-anhydride maléique. Le copolymère méthylvinyléther - anhydride maléique donne des signaux plus forts (courbe AMVE) que le copolymère styrèneanhydride maléique (courbe SAM), toute chose étant égale par ailleurs (concentration en anticorps au moment du couplage, concentration en biotine sur le polymère).
Plus hydrophile que le copolymère styrèneanhydride maléique, le copolymère méthylvinyléther-anhydride maléique conserve mieux l'intégrité de l'anticorps en ne provoquant qu'une faible dénaturation.

**EXEMPLE 7**

Utilisation d'un composé hydrosoluble dérivé du copolymère anhydride maléique/méthylvinyléther, fonctionnalisé par la biotine, comme amplificateur chimique

Dans divers phénomènes biologiques, et tout particulièrement dans le domaine du diagnostic, pour atteindre des niveaux de sensibilité élevés, il peut être nécessaire d'amplifier artificiellement un signal spécifique (par exemple : détection de matériel biologique présent en faible quantité tel que des ADN ou fragments d'ADN).
Dans ce but, des quantités croissantes de biotine-hydrazide (Pierce) ont été mises en réaction avec le copolymère anhydride maléique/méthylvinyléther, C, de l'Exemple 2 pour obtenir des copolymères fonctionnalisés. Ces derniers ont été traités par l'éthanolamine pour conduire à des composés fonctionnalisés hydrosolubles que l'on appelle poly-biotine.

Dans des puits d'une plaque de microtitration (NUNC), on fixe une quantité constante des diverses poly-biotines, par l'intermédiaire d'un anticorps antibiotine, immobilisé au fond des puits selon la méthode décrite dans l'exemple 5. La présence de la biotine est révélée par un conjugué streptavidine - peroxydase qui se fixe en formant un complexe avec la biotine.

La détection, à l'aide de la streptavidine marquée, diluée 1/10 000 en PBS - gélatine 0,5 %°, s'effectue de la façon suivante :

- incubation du conjugué streptavidine-peroxydase pendant une heure à 37°C ;
- 3 lavages en PBS Tween ;
- ajout du substrat orthophénylènediamine (OPD), blocage par $H_2SO_4$ et lecture à 492 nm.

La figure 2 montre le signal obtenu en fonction des quantités de biotine fixée sur le poly(méthylvinyléther- anhydride maléique). On observe qu'il y a une amplification du signal par un facteur 6 environ quand on passe de 0,05 mg de biotine à 1,5 mg de biotine pour 15 mg de polymère.

**Revendications**

1. Composé hydrosoluble dérivé d'un homopolymère ou copolymère de l'anhydride maléique présentant des fonctions anhydride disponibles et des fonctions anhydride hydrolysées, <u>caractérisé en ce que</u> les fonctions anhydride hydrolysées consistent en des fonctions carboxyliques et des fonctions dérivées de fonctions carboxyliques portant un résidu d'un composé répondant à la formule I :

$$Cx \ Hy \ Az \ Ot \qquad\qquad (I)$$

dans laquelle :

- A est l'atome d'azote d'une fonction amine primaire, secondaire ou tertiaire ou l'atome de soufre d'une fonction thiol,
- x, z et t indépendamment les uns des autres sont des nombres entiers non nuls, et
- y est un nombre entier non nul, au moins égal à 5 quand A est un atome d'azote, et au moins égal à 4 quand A est un atome de soufre.

2. Composé selon la revendication 1, caractérisé en ce que A est l'atome d'azote et le composé I est choisi parmi le tris(hydroxyméthyl)aminométhane, l'(amino-2-éthylamino)-2-éthanol, l'amino-2-méthyl-2-propanediol-1,3, la diéthanolamine, les polypeptides et notamment polytyrosine ou polyglycine, le N-BOC-1,6-diaminohexane et les composés répondant aux formules générales respectivement :

$$(II) \qquad HO\text{-}(CH_2)n\text{-}NH_2$$

$$(III) \qquad HO\text{-}(CH_2\text{-}CH_2)n\text{-}O\text{-}(CH_2\text{-}CH_2)n\text{-}NH_2$$

$$(IV) \qquad HO\text{-}(CH_2\text{-}CH_2\text{-}O)n\text{-}CH_2\text{-}CH_2\text{-}NH_2$$

dans lesquelles n est un nombre entier compris entre 1 et 50.

3. Composé selon la revendication 2, caractérisé en ce que n est compris entre 1 et 10 et le composé II est choisi parmi l'éthanolamine et l'(amino-2-éthoxy)-2-éthanol.

4. Composé selon la revendication 1, caractérisé en ce que A est l'atome de soufre et le composé I est le mercaptoéthanol.

**5.** Composé selon la revendication 1, caractérisé en ce que les copolymères de l'anhydride maléique sont choisis parmi les poly (anhydride maléique-éthylène), les poly (anhydride maléique-styrène), les poly (anhydride maléique-propylène), les poly (anhydride maléique-méthylvinyléther).

**6.** Composé selon la revendication 5, caractérisé en ce que le copolymère de l'anhydride maléique comprend au moins 5 % de motifs anhydride maléique.

**7.** Utilisation d'un composé tel que défini selon l'une quelconque des revendications 1 à 6 pour immobiliser, directement ou indirectement, par l'intermédiaire des fonctions anhydride disponibles, une ou plusieurs molécules biologiques choisies parmi les protéines, les anticorps, fragments d'anticorps ou antigènes, les polypeptides, les enzymes, les haptènes, les fragments d'acides nucléiques.

**8.** Utilisation selon la revendication 7 pour immobiliser indirectement une ou plusieurs desdites molécules biologiques, caractérisée en ce que le composé est fonctionnalisé par interaction des fonctions anhydride disponibles avec des fonctions réactives d'un agent de fonctionnalisation, ledit agent de fonctionnalisation étant choisi parmi les haptènes, des agents colorants, luminescents, fluorescents ou phosphorescents et des polymères naturels ou synthétiques notamment polypeptides, polysaccharides et protéines solubles en milieu organique, et le N-BOC-1,6-diaminohexane.

**9.** Utilisation selon la revendication 7 ou 8, caractérisée en ce que le composé est immobilisé directement ou indirectement sur un support solide.

**10.** Utilisation selon la revendication 9, caractérisé en ce que le composé est immobilisé directement sur un support solide par un ligand choisi parmi la polytyrosine, la polyglycine et le N-BOC-1,6-diaminohexane.

**11.** Utilisation selon la revendication 9, caractérisé en ce que le composé est immobilisé indirectement sur un support solide, le composé étant lié à un ligand lui-même lié à un anti-ligand, ce dernier étant lié à un support solide.

**12.** Utilisation selon la revendication 10, caractérisé en ce que le composé répondant à la formule I est à la fois l'agent hydrophilisant et le ligand.

**13.** Utilisation selon la revendication 12, caractérisé en ce que l'agent hydrophilisant est choisi parmi la polytyrosine, la polyglycine et le N-BOC-1,6-diaminohexane.

**14.** Utilisation d'un composé répondant à la formule I :

$$Cx\ Hy\ Az\ Ot \qquad\qquad (I)$$

dans laquelle :

- A est l'atome d'azote d'une fonction amine primaire, secondaire ou tertiaire ou l'atome de soufre d'une fonction thiol,
- x, z et t indépendamment les uns des autres sont des nombres entiers non nuls, et
- y est un nombre entier non nul, au moins égal à 5 quand A est un atome d'azote, et au moins égal à 4 quand A est un atome de soufre, pour hydrophiliser un copolymère ou homopolymère d'anhydride maléique.

**15.** Procédé pour obtenir un dérivé soluble en phase aqueuse à pH neutre, d'un homopolymère ou copolymère d'anhydride maléique caractérisé en ce que :

- on dissout l'homopolymère ou copolymère dans un solvant organique anhydre et
- on fait réagir partiellement les fonctions anhydride de l'homopolymère ou copolymère en solution, avec un agent hydrophilisant répondant à la formule I :

$$Cx\ Hy\ Az\ Ot \qquad\qquad (I)$$

dans laquelle :

- A est l'atome d'azote d'une fonction amine primaire, secondaire ou tertiaire ou l'atome de soufre d'une fonction thiol,
- x, z et t indépendamment les uns des autres sont des nombres entiers non nuls, et
- y est un nombre entier non nul, au moins égal à 5 quand A est un atome d'azote, et au moins égal à 4 quand A est un atome de soufre, en phase organique.

16. Procédé pour immobiliser une molécule biologique sur un homopolymère ou copolymère de l'anhydride maléique caractérisé en ce que :

- on dissout l'homopolymère ou copolymère dans un solvant organique anhydre, et
- on fait réagir partiellement les fonctions anhydride de l'homopolymère ou copolymère dissous avec un agent hydrophilisant répondant à la formule I :

$$Cx \; Hy \; Az \; Ot \qquad\qquad (I)$$

dans laquelle :

- A est l'atome d'azote d'une fonction amine primaire, secondaire ou tertiaire ou l'atome de soufre d'une fonction thiol,
- x, z et t indépendamment les uns des autres sont des nombres entiers non nuls, et
- y est un nombre entier non nul, au moins égal à 5 quand A est un atome d'azote, et au moins égal à 4 quand A est un atome de soufre, en phase organique
- on met en contact le composé hydrosoluble obtenu avec la molécule biologique, en phase aqueuse.

17. Procédé selon la revendication 16, caractérisé en ce qu'avant la mise en contact du composé hydrosoluble avec la molécule biologique, on fonctionnalise l'homopolymère ou copolymère d'anhydride maléique au moyen d'un agent de fonctionnalisation, en phase organique.

18. Procédé selon la revendication 16, caractérisé en ce que la molécule biologique est liée à un agent de fonctionnalisation.


**Patentansprüche**

1. Wasserlösliche Verbindung, derivatisiert aus einem Homopolymeren oder Copolymeren des Maleinsäureanhydrids, die freie Anhydridfunktionen und hydrolisierte Anhydridfunktionen aufweist, <u>dadurch gekennzeichnet,</u> daß die hydrolisierten Anhydridfunktionen aus Carboxylfunktionen und aus von Carboxylfunktionen derivatisierten Funktionen bestehen, die einen Rest einer Verbindung tragen, der der nachfolgenden Formel (I) entspricht:

$$Cx \; Hy \; Az \; Ot \qquad\qquad (I)$$

in der:

- A das Stickstoffatom einer primären, sekundären oder tertiären Aminfunktion oder das Schwefelatom einer Thiolfunktion ist,
- x, z und t, unabhängig voneinander, ganze Zahlen, jedoch nicht Null bedeuten, und
- y eine ganze Zahl, jedoch nicht Null ist, welche, falls A ein Stickstoffatom ist, zumindest gleich 5 ist und, falls A ein Schwefelatom ist, zumindest gleich 4 ist.

2. Verbindung nach Anspruch 1, dadurch gekennzeichnet, daß A das Stickstoffatom ist, und daß die Verbindung (I) ausgewählt ist aus tris(Hydroxymethyl)aminomethan, (Amino-2-ethylamino)-2-ethanol, Amino-2-methyl-2-propandiol-1,3, Diethanolamin, den Polypeptiden und insbesondere Polytyrosin und Polyglycin, N-BOC-1,6-diaminohexan und den Verbindungen, die jeweils den allgemeinen nachfolgenden Formeln entsprechen:

$$(II) \qquad HO\text{-}(CH_2)n\text{-}NH_2$$

(III)     $HO\text{-}(CH_2\text{-}CH_2)n\text{-}O\text{-}(CH_2\text{-}CH_2)n\text{-}NH_2$

(IV)     $HO\text{-}(CH_2\text{-}CH_2\text{-}O)n\text{-}CH_2\text{-}CH_2\text{-}NH_2$

in denen n eine ganze Zahl im Bereich von 1 bis 50 ist.

3.  Verbindung nach Anspruch 2, dadurch gekennzeichnet, daß n im Bereich von 1 bis 10 liegt, und daß die Verbindung (II) ausgewählt ist aus Ethanolamin und (Amino-2-ethoxy)-2-ethanol.

4.  Verbindung nach Anspruch 1, dadurch gekennzeichnet, daß A das Schwefelatom ist, und daß die Verbindung (I) Mercaptoethanol ist.

5.  Verbindung nach Anspruch 1, dadurch gekennzeichnet, daß die Copolymeren des Maleinsäureanhydrids ausgewählt sind aus den Poly-Maleinsäureanhydrid-Ethylenen, den Poly-Maleinsäureanhydrid-Styrolen, den Poly-Maleinsäureanhydrid-Polypropylenen, den Poly-Maleinsäureanhydrid-Methylvinylethern.

6.  Verbindung nach Anspruch 5, dadurch gekennzeichnet, daß das Copolymere des Maleinsäureanhydrids zumindest 5 % Maleinsäureanhydrideinheiten enthält.

7.  Verwendung einer Verbindung nach einem der Ansprüche 1 bis 6 zum direkten oder indirekten Immobilisieren eines oder mehrerer biologischer Moleküle über die freien Anhydridfunktionen, wobei das oder die biologischen Moleküle ausgewählt sind aus den Proteinen, den Antikörpern, Fragmenten von Antikörpern oder Antigenen, den Polypeptiden, den Enzymen, den Haptenen, und den Fragmenten von Nucleinsäuren.

8.  Verwendung nach Anspruch 7 zum indirekten Immobilisieren einer oder mehrerer der genannten biologischen Moleküle, dadurch gekennzeichnet, daß die Verbindung durch Wechselwirkung der freien Anhydridfunktionen mit reaktiven Funktionen eines Funktionalisierungsagens funktionalisiert ist, wobei das Funktionalisierungsagens ausgewählt ist aus den Haptenen, den farbigen, lumineszierenden, fluoreszierenden oder phosphoreszierenden Agenzien, den synthetischen oder natürlichen Polymeren, den insbesondere in organischem Millieu löslichen Polypeptiden, Polysacchariden und Proteinen, und N-BOC-1,6-diaminohexan.

9.  Verwendung nach Anspruch 7 oder 8, dadurch gekennzeichnet, daß die Verbindung direkt oder indirekt auf einem festen Träger immobilisiert ist.

10.  Verwendung nach Anspruch 9, dadurch gekennzeichnet, daß die Verbindung direkt auf einem festen Träger mittels eines Liganden, ausgewählt aus Polytyrosin, Polyglycin und N-BOC-1,6-diaminohexan, immobilisiert ist.

11.  Verwendung nach Anspruch 9, dadurch gekennzeichnet, daß die Verbindung indirekt auf einem festen Träger immobilisiert ist, wobei die Verbindung an einen Liganden gebunden ist, der selbst an einen Antiliganden gebunden ist, wobei letzterer an einen festen Träger gebunden ist.

12.  Verwendung nach Anspruch 10, dadurch gekennzeichnet, daß die Verbindung, die der Formel (I) entspricht, zugleich das hydrophilisierende Agens und der Ligand ist.

13.  Verwendung nach Anspruch 12, dadurch gekennzeichnet, daß das hydrophilisierende Agens ausgewählt ist aus Polytyrosin, Polyglycin und N-BOC-1,6-diaminohexan.

14.  Verwendung einer Verbindung, die der Formel (I) entspricht:

Cx Hy Az Ot     (I)

in der:

-   A das Stickstoffatom einer primären, sekundären oder tertiären Aminfunktion oder das Schwefelatom einer Thiolfunktion ist,

- x, z und t unabhängig voneinander ganze Zahlen, jedoch nicht Null bedeuten, und
- y eine ganze Zahl, jedoch nicht Null ist, welche, falls A ein Stickstoffatom ist, zumindest gleich 5 ist, und falls A ein Schwefelatom ist, zumindest gleich 4 ist, zum Hydrophilisieren eines Copolymeren oder eines Homopolymerens von Maleinsäureanhydrid.

15. Verfahren zur Herstellung eines in wässriger Phase bei neutralem pH löslichen Derivats eines Homopolymeren oder eines Copolymeren von Maleinsäureanhydrid, dadurch gekennzeichnet,

daß das Homopolymere oder Copolymere in einem anhydridischen organischen Lösungsmittel gelöst wird und daß man partiell die Anhydridfunktionen des sich in Lösung befindlichen Homopolymeren oder Copolymeren mit einem hydrophilisierenden Agens, das der Formel (I) entspricht:

$$Cx \; Hy \; Az \; Ot \hspace{8cm} (I)$$

in der:

- A das Stickstoffatom einer primären, sekundären und tertiären Aminfunktion oder das Schwefelatom einer Thiolfunktion ist,
- x, z und t unabhängig voneinander ganze Zahlen, jedoch nicht Null bedeuten, und
- y eine ganze Zahl, jedoch nicht Null ist, welche, falls A ein Stickstoffatom ist, zumindest gleich 5 ist und, falls A ein Schwefelatom ist, zumindest gleich 4 ist, in organischer Phase reagieren läßt.

16. Verfahren zum Immobilisieren eines biologischen Moleküls auf einem Homopolymeren oder einem Copolymeren von Maleinsäureanhydrid, dadurch gekennzeichnet,

daß das Homopolymere oder das Copolymere in einem anhydridischen organischen Lösungsmittel gelöst wird, und
daß man partiell die Anhydridfunktionen des gelösten Homopolymeren oder Copolymeren mit einem hydrophilisierenden Agens, das der Formel (I) entspricht:

$$Cx \; Hx \; Az \; Ot \hspace{8cm} (I)$$

in der:

- A das Stickstoffatom einer primären, sekundären oder tertiären Aminfunktion oder das Schwefelatom einer Thiolfunktion ist,
- x, z und t unabhängig voneinander ganze Zahlen, jedoch nicht Null bedeuten und
- y eine ganze Zahl, jedoch nicht Null ist, welche, falls A ein Stickstoffatom ist, zumindest gleich 5 ist und, falls A ein Schwefelatom ist, zumindest gleich 4 ist, in organischer Phase reagieren läßt, und
- daß man die erhaltene wasserlösliche Verbindung in wässriger Phase mit einem biologischen Molekül in Kontakt bringt.

17. Verfahren nach Anspruch 16, dadurch gekennzeichnet, daß, bevor die wasserlösliche Verbindung mit dem biologischen Molekül in Kontakt gebracht wird, das Homopolymere oder Copolymere des Maleinsäureanhydrids mittels eines Funktionalisierungsagens in organischer Phase funktionalisiert wird.

18. Verfahren nach Anspruch 16, dadurch gekennzeichnet, daß das biologische Molekül an ein Funktionalisierungsagens gebunden ist.

## Claims

1. Water-soluble compound derived from a homopolymer or copolymer of maleic anhydride having available anhydride functional groups and hydrolysed anhydride functional groups, <u>characterized in that</u> the hydrolysed anhydride functional groups consist of carboxyl functional groups and functional groups derived from carboxyl functional groups carrying a residue of a compound corresponding to the formula I:

EP 0 561 722 B1

$$Cx\ Hy\ Az\ Ot \hspace{6cm} (I)$$

in which:

- A is the nitrogen atom of a primary, secondary or tertiary amine functional group or the sulphur atom of a thiol functional group,
- x, z and t, independently of each other, are non-zero integers, and
- y is a non-zero integer, not less than 5 when A is a nitrogen atom and not less than 4 when A is a sulphur atom.

2. Compound according to Claim 1, characterized in that A is the nitrogen atom and the compound I is chosen from tris(hydroxymethyl)aminomethane, 2-(2-aminoethylamino)-ethanol, 2-amino-2-methyl-1,3-propanediol, diethanolamine, the polypeptides and especially polytyrosine and polyglycine, N-BOC-1,6-diaminohexane and the compounds corresponding to the general formulae, respectively:

$$(II) \qquad HO\text{-}(CH_2)n\text{-}NH_2$$

$$(III) \qquad HO\text{-}(CH_2\text{-}CH_2)n\text{-}O\text{-}(CH_2\text{-}CH_2)n\text{-}NH_2$$

$$(IV) \qquad HO\text{-}(CH_2\text{-}CH_2\text{-}O)n\text{-}CH_2\text{-}CH_2\text{-}NH_2$$

in which n is an integer between 1 and 50.

3. Compound according to Claim 2, characterized in that n is between 1 and 10 and the compound II is chosen from ethanolamine and 2-(2-aminoethoxy)ethanol.

4. Compound according to Claim 1, characterized in that A is the sulphur atom and the compound I is is mercaptoethanol.

5. Compound according to Claim 1, characterized in that the copolymers of maleic anhydride are chosen from poly (maleic anhydride-ethylene), poly(maleic anhydride-styrene), poly(maleic anhydride-propylene) or poly(maleic anhydride-methyl vinyl ether).

6. Compound according to Claim 5, characterized in that the copolymer of maleic anhydride comprises at least 5 % of maleic anhydride units.

7. Use of a compound as defined according to any one of Claims 1 to 6 for immobilizing, directly or indirectly, via available anhydride functional groups, one or a number of biological molecules chosen from the proteins, antibodies, fragments of antibodies or antigens, polypeptides, enzymes, haptenes or fragments of nucleic acids.

8. Use according to Claim 7 for indirectly immobilizing one or a number of the said biological molecules, characterized in that the compound is functionalized by interaction of the available anhydride functional groups with reactive functional groups of a functionalization agent, the said functionalization agent being chosen from the haptenes, colouring agents, luminescent agents, fluorescent agents or phosphorescent agents and natural or synthetic polymers, especially polypeptides, polysaccharides and proteins which are soluble in organic medium, and N-BOC-1,6-diaminohexane.

9. Use according to Claim 7 or 8, characterized in that the compound is immobilized directly or indirectly on a solid support.

10. Use according to Claim 9, characterized in that the compound is immobilized directly on a solid support by a ligand chosen from polytyrosine, polyglycine and N-BOC-1,6-diaminohexane.

11. Use according to Claim 9, characterized in that the compound is immobilized indirectly on a solid support, the compound being bonded to a ligand itself bonded to an antiligand, the latter being bonded to a solid support.

14

12. Use according to Claim 10, characterized in that the compound corresponding to the formula I is both the hydrophilizing agent and the ligand.

13. Use according to Claim 12, characterized in that the hydrophilizing agent is chosen from polytyrosine, polyglycine and N-BOC-1,6-diaminohexane.

14. Use of a compound corresponding to the formula I:

$$C_x \, H_y \, A_z \, O_t \tag{I}$$

in which:

-   A is the nitrogen atom of a primary, secondary or tertiary amine functional group or the sulphur atom of a thiol functional group,
-   x, z and t, independently of each other, are non-zero integers, and
-   y is a non-zero integer, not less than 5 when A is a nitrogen atom and not less than 4 when A is a sulphur atom, for hydrophilizing a copolymer or homopolymer of maleic anhydride.

15. Process for producing a derivative, soluble in the aqueous phase at neutral pH, of a homopolymer or copolymer of maleic anhydride, characterized in that:

-   the homopolymer or copolymer is dissolved in an anhydrous organic solvent and
-   the anhydride functional groups of the homopolymer or copolymer in solution are partially reacted with a hydrophilizing agent corresponding to the formula I:

$$C_x \, H_y \, A_z \, O_t \tag{I}$$

in which:

-   A is the nitrogen atom of a primary, secondary or tertiary amine functional group or the sulphur atom of a thiol functional group,
-   x, z and t, independently of each other, are non-zero integers, and
-   y is a non-zero integer, not less than 5 when A is a nitrogen atom and not less than 4 when A is a sulphur atom, in the organic phase.

16. Process for immobilizing a biological molecule on a homopolymer or copolymer of maleic anhydride, characterized in that:

-   the homopolymer or copolymer is dissolved in an anhydrous organic solvent, and
-   the anhydride functional groups of the dissolved homopolymer or copolymer are partially reacted with a hydrophilizing agent corresponding to the formula I:

$$C_x \, H_y \, A_z \, O_t \tag{I}$$

in which:

-   A is the nitrogen atom of a primary, secondary or tertiary amine functional group or the sulphur atom of a thiol functional group,
-   x, z and t, independently of each other, are non-zero integers, and
-   y is a non-zero integer, not less than 5 when A is a nitrogen atom and not less than 4 when A is a sulphur atom, in the organic phase
-   the water-soluble compound obtained is brought into contact with the biological molecule, in the aqueous phase.

17. Process according to Claim 16, characterized in that, before bringing the water-soluble compound into contact with the biological molecule, the homopolymer or copolymer of maleic anhydride is functionalized using a functionalization agent, in the organic phase.

18. Process according to Claim 16, characterized in that the biological molecule is bonded to a functionalization agent.

FIG 1

FIG 2